# EUROPEAN PATENT APPLICATION

(11) **EP 3 503 114 A1**
(43) Date of publication of application: **26.06.2019**
(21) Application number: 17210251.9
(22) Date of filing: 22.12.2017
(51) Int. Cl.: G16H 40/63, G16H 50/30, G16H 50/20

(54) **APPARATUS AND METHOD FOR DETECTING AN ONGOING ISCHEMIC STROKE IN A SUBJECT OR DETECTING WHETHER A SUBJECT IS AT RISK OF DEVELOPING AN ISCHEMIC STROKE**

(71) Applicant: Koninklijke Philips N.V., 5656 AE Eindhoven (NL)
(72) Inventor: VAN LIESHOUT, Ron Martinus Laurentius, 5656 AE Eindhoven (NL); VAN EE, Raymond, 5656 AE Eindhoven (NL)
(74) Representative: de Haan, Poul Erik

(57) **Abstract**

There is provided an apparatus for detecting an ongoing ischemic stroke in a subject or detecting whether the subject is at risk of developing an ischemic stroke, the apparatus comprising a processing unit configured to control a stimulus component to apply a sensory stimulation to the subject to affect the flow of blood to the left part and/or right part of the brain of the subject; obtain measurements of the blood flow in left and right parts of the head and/or neck of the subject for a time period during and/or after the sensory stimulation is applied to the subject; determine a measure of asymmetry in the blood flow in the left and right parts of the head and/or neck of the subject from the obtained measurements; and determine whether the subject has an ongoing ischemic stroke or whether the subject is at risk of developing an ischemic stroke from the determined measure of asymmetry. A corresponding method of operating an apparatus is also provided.

## Description

### FIELD OF THE INVENTION

The present disclosure relates to strokes, and in particular to an apparatus and method for detecting an ongoing ischemic stroke or detecting whether a subject is at risk of developing an ischemic stroke.

### BACKGROUND OF THE INVENTION

A stroke is a life-threatening medical condition caused by a disruption to the blood supply to the brain, thereby resulting in cell death. The most common type of stroke is an ischemic stroke, where the stroke is due to a reduction or lack of blood flow to part of the brain, which can be caused, for instance, by a clot in a blood vessel. Another type of stroke is a haemorrhagic stroke in which the stroke is caused by bleeding in the brain, for example due to a blood vessel rupturing.

Readily perceptible symptoms of a stroke include one side of the subject's face drooping (particularly their mouth), slurring of their speech, and the inability or difficulty to lift both arms up (e.g. by involuntarily allowing one arm to drift downward).

Strokes are a relatively common occurrence. In the US, a stroke occurs on average every 40 seconds, with around 800,000 strokes occurring per year. Strokes can be mild, in which few or mild symptoms occur and there is limited long-lasting impairment to the subject, they can be severe, which can lead to long-lasting or permanent physical and cognitive impairments for the subject (such as vision loss or paralysis), or they can be fatal.

According to current medical guidelines, a subject should receive antiplatelet/anticoagulant medication when having an acute ischemic stroke (e.g. that is caused by a clot obstruction) and medication that encourages coagulation when having a haemorrhagic stroke (e.g. caused by bleeding). Clearly, antiplatelet/anticoagulant medication should not be used in the event that the subject is suffering a haemorrhagic stroke (since the antiplatelet/anticoagulant medication will prevent clotting at the site of the bleeding), and medication that encourages coagulation should not be used in the event that the subject is suffering an ischemic stroke (since the medication that encourages coagulation will potentially worsen the clot or encourage other clots to form).

Since a stroke limits the oxygen supply to parts of the brain, early detection of a stroke and the type of stroke such as to enable the providing of the appropriate treatment is of vital importance. The best way to determine the stroke type is by imaging of the brain (e.g. by means of a CT scan) to assess the type and amount of damage, however this assessment requires specialised equipment that is often only available in a clinical setting (e.g. hospital), and by the time such assessment is made available, irreparable damages can have been caused.

WO 2016/178797 describes that a cardiovascular function can be assessed using an optical sensor that can sense haemodynamics, such as skin colour and skin and other organ displacement. This document also describes that an asymmetry in blood flow within a patient's face can be determined, and asymmetry can indicate a stroke or other vascular disease.

Although WO 2016/178797 suggests that an asymmetry in blood flow within a patient's face can be determined, it has been found that often the obtained blood flow signal is not good enough to enable a reliable measure of asymmetry to be determined.

Currently therefore it is not possible to reliably determine whether a subject is suffering from an ischemic or haemorrhagic strokes quickly and in an unobtrusive way.

### SUMMARY OF THE INVENTION

There is therefore a need for improvements in the detection of an ongoing ischemic stroke in a subject. There is also a need for ways to detecting whether a subject is at risk of developing an ischemic stroke.

According to a first specific aspect, there is provided an apparatus for detecting an ongoing ischemic stroke in a subject or detecting whether the subject is at risk of developing an ischemic stroke, the apparatus comprising a processing unit configured to control a stimulus component to apply a sensory stimulation to the subject to affect the flow of blood to the left part and/or right part of the brain of the subject; obtain measurements of the blood flow in left and right parts of the head and/or neck of the subject for a time period during and/or after the sensory stimulation is applied to the subject; determine a measure of asymmetry in the blood flow in the left and right parts of the head and/or neck of the subject from the obtained measurements; and determine whether the subject has an ongoing ischemic stroke or whether the subject is at risk of developing an ischemic stroke from the determined measure of asymmetry. Thus, this aspect provides that any asymmetry in the blood flow in the head and/or neck of the subject can be enhanced, which improves the reliability of the detection of an ongoing ischemic stroke and the detection of the subject being at an increased risk of ischemic stroke.

In some embodiments, the processing unit is configured to determine from the measure of asymmetry whether the subject has an ongoing ischemic stroke or is at risk of developing an ischemic stroke by comparing the measure of asymmetry to a threshold value. The threshold value can be set to a value that allows for a small amount of asymmetry before an ischemic stroke is detected or before detecting that the subject is at risk of an ischemic stroke, thus the reliability of the detection can be improved.

In some embodiments, the processing unit is configured to determine from the measure of asymmetry whether the subject has an ongoing ischemic stroke or is at risk of developing an ischemic stroke by comparing the determined measure of asymmetry to a previous measure of asymmetry for the subject or to a measure of asymmetry for a population of subjects. These embodiments provide that changes in the asymmetry for the subject over time, or relative to a population of subjects, can be used to detect whether the subject has an ongoing ischemic stroke or detect whether the subject is at risk of ischemic stroke.

In some embodiments, the processing unit is further configured to provide an output indicating whether the subject has an ongoing ischemic stroke or is at risk of developing an ischemic stroke. This output can be used by the subject to inform a care provider (e.g. clinician or paramedic) of their current condition, and/or used by a care provider to provide the subject with an appropriate treatment.

In some embodiments, the processing unit is further configured to determine that the subject has an ongoing haemorrhagic stroke if it is determined that the subject does not have an ongoing ischemic stroke. These embodiments are useful as haemorrhagic strokes require different treatment to ischemic strokes.

In some embodiments, the processing unit is configured to determine the measure of the asymmetry in the blood flow in the left and right parts of the head and/or neck of the subject by determining a measure of the blood flow in the left part of the head and/or neck of the subject; determining a measure of the blood flow in the right part of the head and/or neck of the subject; and determining the measure of the asymmetry as a difference between, or a ratio of, the determined measure of the blood flow in the left part and the determined measure of the blood flow in the right part.

In some embodiments, the processing unit is further configured to obtain measurements of the blood flow in left and right parts of the head and/or neck of the subject for a time period before the sensory stimulation is applied to the subject; and normalise the measurements of the blood flow in left and right parts of the head and/or neck of the subject for the time period during and/or after the sensory stimulation is applied using the measurements of the blood flow in left and right parts of the head and/or neck of the subject for the time period before the sensory stimulation is applied; wherein the processing unit is configured to determine the measure of asymmetry from the normalised measurements of blood flow. These embodiments are useful as they help to enhance the effect of the stimulus on the measure of asymmetry, and thus improve the reliability of the indication of whether the subject has an ongoing ischemic stroke or whether the subject is at an increased risk of ischemic stroke.

In some embodiments, the processing unit is further configured to control the stimulus component to apply a second sensory stimulation to the subject to affect the flow of blood to the left part and/or right part of the brain of the subject; obtain measurements of the blood flow in left and right parts of the head and/or neck of the subject for a time period during and/or after the second sensory stimulation is applied to the subject; and determine a second measure of asymmetry in the blood flow in the left and right parts of the head and/or neck of the subject from the measurements for the time period during and/or after the second sensory stimulation is applied; wherein the processing unit is configured to determine whether the subject has an ongoing ischemic stroke or whether the subject is at risk of developing an ischemic stroke from the determined measure of asymmetry and the determined second measure of asymmetry. These embodiments enable continuous, near continuous, or regular monitoring of the subject over time to enable an ischemic stroke or an increased risk of an ischemic stroke to be detected as early as possible.

In these embodiments, the processing unit can be configured to determine whether the subject has an ongoing ischemic stroke or is at risk of developing an ischemic stroke based on a difference between the determined measures of asymmetry.

In some embodiments, the sensory stimulation affects the flow of blood to one of the parts of the brain of the subject, and wherein the second sensory stimulation affects the flow of blood to the other part or both parts of the brain of the subject. These embodiments are useful as a sensory stimulus to one side of the brain may not produce an effect on the blood flow of the subject if they have a blockage/clot on that side of the head and/or neck (i.e. any measure of asymmetry will not be enhanced by the stimulus), but applying a sensory stimulus to the other side of the brain may provide an enhanced measure of asymmetry since that side of the head and/or neck does not have a blockage/clot.

According to a second aspect, there is provided a system for detecting an ongoing ischemic stroke in a subject or detecting whether the subject is at risk of developing an ischemic stroke, the system comprising an apparatus according to the first aspect or any embodiment thereof, and one or both of the stimulus component for applying a sensory stimulation to the subject, and a blood flow measurement device for measuring the blood flow in the left part and right part of the head and/or neck of the subject.

Alternatively, the system for detecting an ongoing ischemic stroke in a subject or detecting whether the subject is at risk of developing an ischemic stroke comprises the apparatus according to the first aspect or any embodiment thereof, one or both of the stimulus component for applying a sensory stimulation to the subject, or a blood flow measurement device for measuring the blood flow in the left part and right part of the head and/or neck of the subject. According to a third specific aspect, there is provided a method of operating an apparatus to detect an ongoing ischemic stroke in a subject or detect whether the subject is at risk of developing an ischemic stroke the method comprising controlling a stimulus component to apply a sensory stimulation to the subject to affect the flow of blood to the left part and/or right part of the brain of the subject; obtaining measurements of the blood flow in left and right parts of the head and/or neck of the subject for a time period during and/or after the sensory stimulation is applied to the subject; determining a measure of asymmetry in the blood flow in the left and right parts of the head and/or neck of the subject from the obtained measurements; and determining whether the subject has an ongoing ischemic stroke or whether the subject is at risk of developing an ischemic stroke from the determined measure of asymmetry. Thus, this aspect provides that any asymmetry in the blood flow in the head and/or neck of the subject can be enhanced, which improves the reliability of the detection of an ongoing ischemic stroke and the detection of the subject being at an increased risk of ischemic stroke.

In some embodiments, the method comprises determining from the measure of asymmetry whether the subject has an ongoing ischemic stroke or is at risk of developing an ischemic stroke by comparing the measure of asymmetry to a threshold value. The threshold value can be set to a value that allows for a small amount of asymmetry before an ischemic stroke is detected or before detecting that the subject is at risk of an ischemic stroke, thus the reliability of the detection can be improved.

In some embodiments, the method comprises determining from the measure of asymmetry whether the subject has an ongoing ischemic stroke or is at risk of developing an ischemic stroke by comparing the determined measure of asymmetry to a previous measure of asymmetry for the subject or to a measure of asymmetry for a population of subjects. These embodiments provide that changes in the asymmetry for the subject over time, or relative to a population of subjects, can be used to detect whether the subject has an ongoing ischemic stroke or detect whether the subject is at risk of ischemic stroke.

In some embodiments, the method further comprises providing an output indicating whether the subject has an ongoing ischemic stroke or is at risk of developing an ischemic stroke. This output can be used by the subject to inform a care provider (e.g. clinician or paramedic) of their current condition, and/or used by a care provider to provide the subject with an appropriate treatment.

In some embodiments, the method further comprises determining that the subject has an ongoing haemorrhagic stroke if it is determined that the subject does not have an ongoing ischemic stroke. These embodiments are useful as haemorrhagic strokes require different treatment to ischemic strokes.

In some embodiments, the method comprises determining the measure of the asymmetry in the blood flow in the left and right parts of the head and/or neck of the subject by determining a measure of the blood flow in the left part of the head and/or neck of the subject; determining a measure of the blood flow in the right part of the head and/or neck of the subject; and determining the measure of the asymmetry as a difference between, or a ratio of, the determined measure of the blood flow in the left part and the determined measure of the blood flow in the right part.

In some embodiments, the method comprises obtaining measurements of the blood flow in left and right parts of the head and/or neck of the subject for a time period before the sensory stimulation is applied to the subject; and normalising the measurements of the blood flow in left and right parts of the head and/or neck of the subject for the time period during and/or after the sensory stimulation is applied using the measurements of the blood flow in left and right parts of the head and/or neck of the subject for the time period before the sensory stimulation is applied; wherein the method comprises determining the measure of asymmetry from the normalised measurements of blood flow. These embodiments are useful as they help to enhance the effect of the stimulus on the measure of asymmetry, and thus improve the reliability of the indication of whether the subject has an ongoing ischemic stroke or whether the subject is at an increased risk of ischemic stroke.

In some embodiments, the method comprises controlling the stimulus component to apply a second sensory stimulation to the subject to affect the flow of blood to the left part and/or right part of the brain of the subject; obtaining measurements of the blood flow in left and right parts of the head and/or neck of the subject for a time period during and/or after the second sensory stimulation is applied to the subject; and determining a second measure of asymmetry in the blood flow in the left and right parts of the head and/or neck of the subject from the measurements for the time period during and/or after the second sensory stimulation is applied; wherein the method comprises determining whether the subject has an ongoing ischemic stroke or whether the subject is at risk of developing an ischemic stroke from the determined measure of asymmetry and the determined second measure of asymmetry. These embodiments enable continuous, near continuous, or regular monitoring of the subject over time to enable an ischemic stroke or an increased risk of an ischemic stroke to be detected as early as possible.

In these embodiments, the method comprises determining whether the subject has an ongoing ischemic stroke or is at risk of developing an ischemic stroke based on a difference between the determined measures of asymmetry.

In some embodiments, the sensory stimulation affects the flow of blood to one of the parts of the brain of the subject, and wherein the second sensory stimulation affects the flow of blood to the other part or both parts of the brain of the subject. These embodiments are useful as a sensory stimulus to one side of the brain may not produce an effect on the blood flow of the subject if they have a blockage/clot on that side of the head and/or neck (i.e. any measure of asymmetry will not be enhanced by the stimulus), but applying a sensory stimulus to the other side of the brain may provide an enhanced measure of asymmetry since that side of the head and/or neck does not have a blockage/clot.

According to a fourth aspect, there is provided a computer program product comprising a computer readable medium having computer readable code embodied therein, the computer readable code being configured such that, on execution by a suitable computer or processor, the computer or processor is caused to perform the method according to the second aspect or any embodiment thereof.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 shows a block diagram of an apparatus according to an aspect of the techniques described herein and a stimulus component; and
Fig. 2 is a flow chart illustrating a method of operating an apparauts according to an aspect of the techniques described herein.

### DETAILED DESCRIPTION OF EMBODIMENTS

As noted above, the present disclosure relates to improvements in the detection of an ongoing ischemic stroke and detecting whether a subject is at risk of developing ischemic stroke. In particular, an apparatus and method are provided that are for detecting an ongoing ischemic stroke in a subject or detecting whether a subject is at risk of developing ischemic stroke. Before providing the details of the apparatus and method, some general information is provided regarding the blood vessel system in the head.

Normal functioning of the brain's control centres is dependent on an adequate supply of oxygen and nutrients through a dense network of blood vessels. Blood is supplied to the brain, face and scalp via two major sets of vessels: the left and right common carotid arteries and the left and right vertebral arteries.

The common carotid arteries have two divisions. The external carotid arteries supply the face and scalp with blood. The internal carotid arteries supply blood to the anterior three-fifths of the cerebrum, except for parts of the temporal and occipital lobes. The vertebrobasilar arteries supply the posterior two-fifths of the cerebrum, part of the cerebellum, and the brain stem.

An obstruction of the internal carotid arteries (e.g. due to a clot) does not necessarily lead to complete disruption of cerebral flow due to cross circulation in the head via the circle of Willis. Alternatively, there can be a redirection of blood from the external carotid arteries to the internal carotid arteries by collateral pathways.

Whenever a clot or other blood obstruction develops in the left (or right) side of the brain, there will be an asymmetry in the amount of blood and oxygen in the left and right parts of the face and neck (e.g. a higher amount of blood and oxygen on one side compared to the other side). The asymmetry of the blood flow in the face is caused by the redirection of the blood via arteries in the face.

The inventors have found that small left-right asymmetry in facial expressions (or more generally asymmetry in the face) and in blood flow that increase over time are signs of a developing ischemic stroke (e.g. due to blood flow in part of the brain being hindered by a clot or plaque). However, this small level of asymmetry is difficult to detect from the normal variation in the blood flow using the techniques described in WO 2016/178797.

In addition, it is difficult (if not impossible in some cases) to diagnose a stroke type based on anatomical and behavioural symptoms (e.g. face drooping, slurred speech, etc.), and a definitive detection using brain imaging technologies takes time. This time will delay the treatment and thus will cause additional damage to the brain tissue. The inventors have found that the blood flow signal obtained using techniques such as those described in WO 2016/178797 is not good enough to enable a reliable measure of asymmetry to be determined.

The techniques described herein make use of measurements of the asymmetry in the blood flow in the head (particularly the face) and/or neck of a subject for unobtrusive detection of an ongoing ischemic stroke or detection of whether a subject is at risk of developing ischemic stroke. To provide more reliable or more useful blood flow measurements, the activity of the brain of the subject is increased by using a sensory stimulus (e.g. sound, light, speech, movement), which increases the blood flow the brain (or the relevant side of the brain), and thus enhances the asymmetry and the difference in blood perfusion in the left and right sides of the head (face) and/or neck, and therefore makes the asymmetry easier to detect. In addition, the use of a stimulus to increase the blood flow in the head and/or neck can allow the measurements of the blood flow and variations in the measurements of the blood flow to be normalised.

Thus, in some embodiments measurements of the blood flow can be taken frequently (for example every day, every hour, every week), and the measurements analysed to determine if an asymmetry is developing in the head and/or neck of the subject. If such an asymmetry is detected, this can be indicative of the subject being at risk of developing ischemic stroke.

If a stroke has occurred or is occurring, measurements of the blood flow can be taken following or during a sensory stimulus to detect whether the subject is suffering from an ischemic stroke. If it is known that the subject is suffering a stroke (without knowing which type), then the techniques described herein can be used to determine whether the stroke is an ischemic stroke, and if not, it can be determined that the stroke is a haemorrhagic stroke. These techniques could be used in a clinical setting (e.g. a hospital), at home, or in transit to a clinical setting (e.g. in an ambulance). In particular, whenever there is a clot in an internal carotid artery then part of the external carotid system takes over the blood supply to the head. This is not the case for broken internal carotid artery (which causes a haemorrhagic stroke). Therefore, by measuring the blood flow through the external carotid system on the left and right sides of the head and/or neck during or following the sensory stimulus, it can be determined whether the ongoing stroke is an ischemic or haemorrhagic stroke.

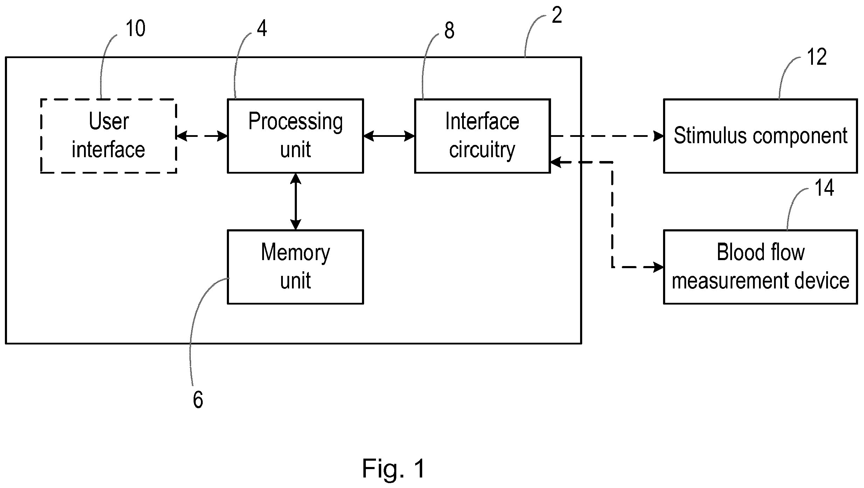

Fig. 1 shows a block diagram of an apparatus 2 that is for detecting an ongoing ischemic stroke in a subject or detecting whether a subject is at risk of developing ischemic stroke according to the techniques described herein. It will be appreciated that by 'at risk of developing ischemic stroke', it is meant a binary decision as to whether the subject has a high (or increased) risk of an ischemic stroke, or a normal/low (or decreased) risk of an ischemic stroke, and not a particular risk score or risk profile.

The apparatus 2 includes a processing unit 4 that controls the operation of the apparatus 2 and that can be configured to execute or perform the methods described herein. The processing unit 4 can be implemented in numerous ways, with software and/or hardware, to perform the various functions described herein. The processing unit 4 may comprise one or more microprocessors or digital signal processor (DSPs) that may be programmed using software or computer program code to perform the required functions and/or to control components of the processing unit 4 to effect the required functions. The processing unit 4 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 4 is connected to a memory unit 6 that can store data, information and/or signals for use by the processing unit 4 in controlling the operation of the apparatus 2 and/or in executing or performing the methods described herein. In some implementations the memory unit 6 stores computer-readable code that can be executed by the processing unit 4 so that the processing unit 4 performs one or more functions, including the methods described herein. The memory unit 6 can comprise any type of non-transitory machine-readable medium, such as cache or system memory including volatile and nonvolatile computer memory such as random access memory (RAM) static RAM (SRAM), dynamic RAM (DRAM), read-only memory (ROM), programmable ROM (PROM), erasable PROM (EPROM), and electrically erasable PROM (EEPROM).

The apparatus 2 also includes interface circuitry 8 for enabling a data connection to and/or data exchange with other devices, including any one or more of servers, databases, user devices, and sensors. The interface circuitry 8 can enable a connection between the apparatus 2 and a network, such as the Internet, via any desirable wired or wireless communication protocol. For example, the interface circuitry 8 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). The interface circuitry 8 is connected to the processing unit 4.

In some embodiments, the apparatus 2 comprises a user interface 10 that includes one or more components that enables a user of apparatus 2 to input information, data and/or commands into the apparatus 2, and/or enables the apparatus 2 to output information or data to the user of the apparatus 2, such as an indication of whether the subject is suffering an ischemic stroke or a haemorrhagic stroke, or a risk of an upcoming stroke. The user interface 10 can comprise any suitable input component(s), including but not limited to a keyboard, keypad, one or more buttons, switches or dials, a mouse, a track pad, a touchscreen, a stylus, a camera, a microphone, etc., and the user interface 10 can comprise any suitable output component(s), including but not limited to a display screen, one or more lights or light elements, one or more loudspeakers, a vibrating element, etc.

The apparatus 2 can be any type of electronic device or computing device. For example the apparatus 2 can be, or be part of, a server, a computer, a laptop, a tablet, a smartphone, smart watch, smart mirror (e.g. for use in a bathroom), etc.

It will be appreciated that a practical implementation of an apparatus 2 may include additional components to those shown in Fig. 1. For example the apparatus 2 may also include a power supply, such as a battery, or components for enabling the apparatus 2 to be connected to a mains power supply.

In accordance with the techniques described herein, a stimulus component 12 is used to provide a sensory stimulus (e.g. sound, light, speech, movement) that aims to affect the flow of blood to the left part and/or right part of the brain of the subject by increasing the activity of the brain of the subject, which enhances any asymmetry and/or difference in blood perfusion in the left and right sides of the head and/or neck of the subject. The stimulus component 12 is shown in Fig. 1 as being separate from the apparatus 2, with the stimulus component 12 being connected to the apparatus 2 via the interface circuitry 8. In alternative embodiments, the stimulus component 12 can be part of the apparatus 2, in which case the stimulus component 12 can be connected directly to the processing unit 4.

In either embodiment, the processing unit 4 can provide a control signal to the stimulus component 12 to cause a stimulus to be provided to the subject at a required time or times. Depending on the type of stimulus component 12, the sensory stimulus provided by the stimulus component 12 may be adjustable or configurable, and the control signal from the processing unit 4 can provide an indication of a setting or parameter for the sensory stimulus so that the stimulation component 12 provides the appropriate sensory stimulus to the subject.

The stimulus component 12 can be any type of component or device that can provide a sensory stimulus such as sound, light, speech, vibration, movement, electrical and/or magnetic stimulation, etc. (or any combination thereof) to a subject. For example the stimulus component 12 can comprise one or more loudspeakers for outputting sound (e.g. speech (particularly speech by someone known to the subject, e.g. parent, child, etc.), a beep, buzz, chirp, music, etc.), one or more light sources for outputting light (e.g. at a particular or selectable colour or range of colours, at a particular or selectable brightness or intensity, etc.), a display screen for providing light, a static visual image or a moving visual image (e.g. video) to the subject, or any combination thereof. It will be appreciated that, where the stimulus component 12 is part of the apparatus 2, depending on the type of sensory stimulus to be provided, the stimulus component 12 may be, or make use of, one or more components in the user interface 10 (e.g. the loudspeaker, display screen, light sources, etc.). In some embodiments, the sensory stimulus can be or include a magnetic signal or electromagnetic signal that is applied to the head and/or neck of the subject to stimulate the brain. The intensity of the stimulation may be controllable. This stimulus can be, for example, transcranial magnetic stimulation (TMS). In some embodiments, the sensory stimulus can be a tactile stimulus, such as a vibration or similar.

In some embodiments, the stimulus component 12 can provide a sensory stimulus that stimulates both sides (i.e. left and right) of the subject's brain equally (or approximately equally). For example the stimulus component 12 could generate a sound that can be heard by both ears of the subject, or generate a light or visual image/video that can be seen by both eyes of the subject. Alternatively, the stimulus component 12 can provide a sensory stimulus that stimulates just one side (i.e. the left or right) of the subject's brain, or that stimulates one side of the subject's brain more than the other side. For example, the stimulus component 12 can generate a sound that can only (or primarily) be heard by one of the ears of the subject (e.g. by outputting the sound through one side of a set of headphones, or otherwise by outputting a sound via a loudspeaker placed closer to one of the subject's ears than the other). As another example, the stimulus component 12 may generate a visual stimulus that can only (or primarily) be seen by one of the eyes of the subject (e.g. by providing the visual stimulus using a virtual reality (VR) device, such as a headset or pair of glasses).

In any of these embodiments, the side of the brain stimulated can be selectable, for example the stimulus component 12 may be able to selectively provide a stimulus to the left side of the brain and/or selectively provide a stimulus to the right side of the brain (and optionally therefore also selectively provide a stimulus to both the left and right sides of the brain at the same time). This selection may be in response to a control signal from the processing unit 4.

In some embodiments, the stimulus component 12 can be configured to provide stimulus of multiple types to increase the effect on the blood flow in the subject. For example the stimulus component 12 can provide a visual stimulus in combination with an audible stimulus, a visual stimulus in combination with a tactile stimulus, a visual stimulus in combination with an audible stimulus and a tactile stimulus, etc. These combinations of stimulus can be provided to stimulate one or both sides of the brain of the subject.

Those skilled in the art will be aware of various other types of stimulus and/or stimulus component 12 that can be used to provide a sensory stimulus to a subject (whether to one or both sides of the brain of the subject).

Fig. 1 also shows a blood flow measurement device 14. The blood flow measurement device 14 is provided to measure the blood flow on the left side of head and/or neck of the subject over time, and to measure the blood flow on the right side of the head and/or neck of the subject over time. The blood flow measurement device 14 may measure the perfusion of the blood in the skin of the subject over time on the left and right sides using any suitable measurement technique, including by measuring the oxygen saturation (also known as SpO2) over time on the left and right sides (in which case the blood flow measurement device 14 can be a photopleythsmogram (PPG) sensor, an SpO2 sensor, or a sensor that uses near infrared (NIR) spectroscopy), measuring blood volume over time on the left and right sides (in which case the blood flow measurement device 14 can be a PPG sensor), measuring blood perfusion over time on the left and right sides (in which case the blood flow measurement device 14 can be laser speckle contrast analysis (LASCA)-based), or by measuring any other indication of blood flow. As is known, LASCA illuminates the skin with laser light, and the backscattered light will form a random interference pattern consisting of dark and bright areas. This pattern is called a speckle pattern. If the illuminated skin is static, the speckle pattern will be stationary. When there is movement in the skin, such as red blood cells, the speckle pattern will change over time.

That is, the blood flow measurement device 14 provides separate measurements of the blood flow on the left and right sides (so a signal/set of measurements for the left side, and a signal/set of measurements for the right side). The blood flow measurement device 14 is shown in Fig. 1 as being separate from the apparatus 2, with the blood flow measurement device 14 being connected to the apparatus 2 via the interface circuitry 8 to enable the measurements to be provided to the processing unit 4 for analysis or processing, or to the memory unit 6 for storage. In alternative embodiments, the blood flow measurement device 14 can be part of the apparatus 2, in which case the blood flow measurement device 14 can be connected directly to the processing unit 4. In either case, the blood flow measurement device 14 may continuously obtain measurements of the blood flow, or it may obtain measurements of the blood flow in response to an activation signal from the processing unit 4.

The blood flow measurement device 14 can be any type of component or device that can provide separate measurements of the blood flow on the left and right sides of the head (face) and/or neck of the subject. In some embodiments, the blood flow measurement device 14 is or comprises one or more cameras that is/are used to obtain images or video of the head (face) and/or neck of the subject, and these images or video are processed to extract blood flow measurements of the left and right sides of the head and/or neck. Those skilled in the art will be aware of various image/video processing techniques that can be used to extract blood flow measurements from images or videos. Such techniques include those relating to remote PPG (rPPG). In other embodiments the blood flow measurement device 14 can comprise a first blood flow sensor that can be used to measure the blood flow on the left side of the subject's head and/or neck and a second blood flow sensor that can be used to measure the blood flow on the right side of the subject's head and/or neck. The first blood flow sensor and the second blood flow sensor may be such that they are placed in contact with or proximate to the skin of the subject on the appropriate side of the head and/or neck to measure the blood flow. Each blood flow sensor may be a PPG sensor, an SpO2 sensor, or any other type of contact-based blood flow sensor. As an example, a blood flow sensor could be placed on each cheek of the subject, on each ear of the subject, on each side of the neck, on each temple, etc. It will be appreciated that it is possible for the first and second blood flow sensors to be placed on different parts of the head and/or neck, for example the first blood flow sensor could be placed on the left ear of the subject and the second blood flow sensor could be placed on the right cheek of the subject.

It will be appreciated that the accuracy of blood flow measurements obtained by processing images or a video sequence obtained using a camera can be affected by the amount of ambient light and the facial veins themselves (i.e. variation in perfusion due to microvascular contraction). The accuracy of other forms of blood flow measurement may also be affected by various factors. An increase in blood flow measured by the blood flow measurement device 14 following the application of the sensory stimulus can be directly correlated with the supply of blood to the brain. For example, when the stimulus component 12 only stimulates one side of the brain (e.g. with speech provided to one ear of the subject), only one side of the face and/or neck will show differences in perfusion and blood oxygen levels. The blood flow measurement obtained for the other side of the face and/or neck can be used to normalise any variation caused by the ambient light and skin perfusion.

As noted above, the techniques described herein provide that a sensory stimulus is provided by the stimulus component 12 to increase the blood flow to one or both of the left and right sides of the subject's brain, and thus increase any asymmetry in the blood flow between the two sides. When the blood supply in the brain is hampered (e.g. due to a stroke) part of the blood supply to the face is redirected to supply the brain. The primary route of redirection will follow a blood vessel circuit that is called the "circle of Willis". Thus, the sensory stimulus increases the amount of blood redirected from the facial veins to the brain. Asymmetry in the blood flow following the stimulus can be measured using the blood flow measurement device 14 and an ischemic stroke detected based on the asymmetry present in the blood flow measurements. If a stroke is known to be occurring, then a lack of asymmetry in the blood flow measurements can indicate that the subject is suffering a haemorrhagic stroke. If the stimulus is applied and measurements of blood flow made over the course of several days (for example), an observed increase in asymmetry in the blood flow can indicate that the subject is at risk of developing an ischemic stroke (or has an ongoing ischemic stroke).

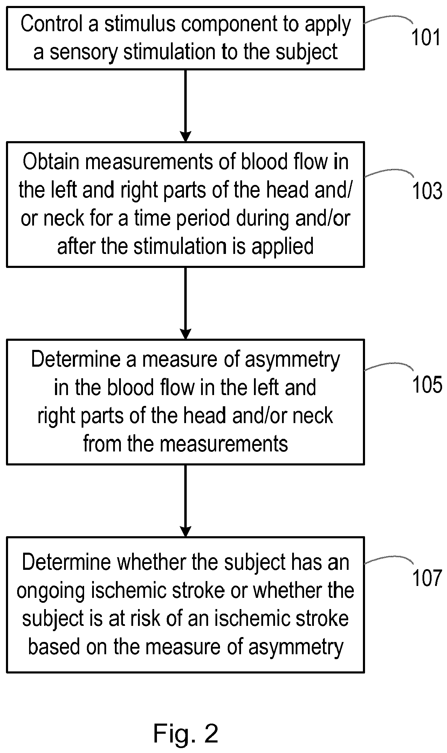

The flow chart in Fig. 2 illustrates a method of detecting an ongoing ischemic stroke in a subject or detecting whether a subject is at risk of developing ischemic stroke according to the techniques described herein. The method can be performed by the apparatus 2, and in particular by the processing unit 4.

In a first step, step 101, a stimulus component 12 is controlled to apply a sensory stimulation to the subject to affect the flow of blood to the left part and/or right part of the brain of the subject. Step 101 can comprise the processing unit 4 providing an appropriate control signal or signals to the stimulus component 12 so that the stimulus component 12 provides the stimulus to the subject.

As noted above, the sensory stimulation may be any type of stimulation (e.g. visual, audible, tactile, etc.), and the sensory stimulation may be provided to one or both sides of the head and/or neck of the subject (e.g. left eye, right eye, left ear, right ear, etc.). The type, content (e.g. speech, sound, display) and/or side(s) that the stimulation is to be applied to can be controlled by the control signal from the processing unit 4. Alternatively, the type and/or side(s) that the stimulation is to be applied to can be preset or fixed (e.g. the stimulus component 12 is only able to provide a stimulus of a particular type and/or content, and/or the stimulus component 12 may only be able to provide the stimulus to both sides of the brain of the subject, or one particular side), in which case the control signal from the processing unit 4 can simply cause the stimulus component 12 to provide the preset or fixed stimulus.

In embodiments where the processing unit 4 is able to control the type, content and/or side(s) that the stimulation is to be applied to, prior to step 101 the method may further comprise the step of determining the type of stimulation to be applied, the content to be used for the stimulation, and/or the side(s) that the stimulation is to be applied, and providing an appropriate control signal in step 101.

In step 103, measurements of the blood flow in left and right parts of the head and/or neck of the subject are obtained. These measurements are for a time period during which the sensory stimulation is applied, and/or for a time period that is after the sensory stimulation was applied. The measurements are obtained using blood flow measurement device 14, and thus step 103 can comprise the processing unit 4 receiving the measurements from the blood flow measurement device 14. Step 103 can also comprise the processing unit 4 activating or otherwise controlling the blood flow measurement device 14 to start measuring blood flow at an appropriate time.

The measurements obtained in step 103 therefore comprise a set of measurements of the blood flow in the left part of the head and/or neck of the subject, and a set of measurements of the blood flow in the right part of the head and/or neck of the subject.

In step 105 (which may occur following receipt of the measurements in step 103, or some time later, as required), a measure of asymmetry in the blood flow in the left and right parts of the head and/or neck of the subject is determined from the obtained measurements. That is, a measure of the asymmetry between the blood flow in the left part of the head and/or neck and the blood flow in the right part of the head and/or neck is determined.

Thus, in some embodiments, the processing unit 4 determines the measure of asymmetry by determining the difference between the measurements of blood flow in the left part of the head and/or neck and the measurements of the blood flow in the right part of the head and/or neck (e.g. by subtracting one set of measurements from the other set). The measure of asymmetry corresponds to this difference. In some embodiments, the measure of asymmetry can be the difference between an average (e.g. mean, mode, median) of the measurements of blood flow in the left part of the head and/or neck and an average (e.g. mean, mode, median) of the measurements of blood flow in the right part of the head and/or neck. It will be appreciated that in either case a difference of 0 indicates no asymmetry, and a non-zero difference indicates asymmetry, with the magnitude of the difference indicating the magnitude of the asymmetry.

Alternatively, the processing unit 4 determines the measure of asymmetry by determining a ratio of the measurements of blood flow in the left part of the head and/or neck and the measurements of the blood flow in the right part of the head and/or neck (e.g. by dividing one set of measurements by the other set). The measure of asymmetry corresponds to this ratio. As above, the measure of asymmetry can be based on an average of the blood flow measurement for the left part and an average of the blood flow measurement for the right part. It will be appreciated that in either case a ratio of 1 indicates no asymmetry, and a value other than 1 indicates asymmetry, with the magnitude of the ratio indicating the magnitude of the asymmetry.

In some embodiments, as the response by the brain to the stimulus may take some time to have an effect on the blood flow in the head and/or neck, several measures of asymmetry can be determined from the measurements of blood flow obtained after the stimulus is applied. For example a first measure of asymmetry can be formed from a set of measurements for a first time period after the stimulus (e.g. the first 5 seconds after the stimulus is applied), a second measure of asymmetry can be formed from a set of measurements for a second time period after the stimulus (e.g. the next 5 seconds after the stimulus is applied), and so on.

As noted above, the application of the sensory stimulation in step 101 will have the effect of increasing any asymmetry present between the left side and right side of the subject's head and/or neck, and thus the asymmetry will be more evident in the measure of asymmetry determined in step 105.

Then, in step 107, the determined measure of asymmetry is used to determine an indication of whether the subject has an ongoing ischemic stroke or whether a subject is at risk of developing ischemic stroke.

In some embodiments step 107 comprises comparing the measure of asymmetry to a threshold value. The threshold value can have any suitable value, and for example, in the embodiments where the measure of asymmetry is formed as a ratio, the threshold value can have two values, e.g. an upper value, such as 1.2, and a lower value, such as 0.8, and it can be determined that the subject has an ongoing ischemic stroke or is at risk of developing ischemic stroke if the measure of asymmetry is larger than the upper threshold value (e.g. 1.2) or lower than the lower threshold value (e.g. 0.8). Where the measure of asymmetry is formed as a difference, the magnitude of the measure of asymmetry can be compared to a threshold value, and it will be appreciated by those skilled in the art that the value of the threshold will depend on the specific blood flow parameter that has been measured (e.g. the blood volume, blood perfusion, PPG, etc.).

In some embodiments step 107 comprises using the determined measure of asymmetry to determine whether the subject has an ongoing ischemic stroke or is at risk of developing an ischemic stroke by comparing the determined measure of asymmetry to a previously-determined measure of asymmetry for the subject (e.g. a measure of asymmetry determined after applying a stimulus according to the techniques described herein) to determine if there has been a change in the asymmetry. If this comparison shows that the asymmetry has increased, then it can be indicated that the subject has an ischemic stroke or is at risk of developing an ischemic stroke. If this comparison shows that the asymmetry has decreased, then it can be indicated that the subject does not have an ischemic stroke or is not at risk of developing an ischemic stroke. Alternatively, the determined measure of asymmetry can be compared to a measure of asymmetry for a population of subjects to determine if the subject has a higher level of asymmetry than the population of subjects. If this comparison shows that the subject has a higher level of asymmetry, then it can be detected that the subject has an ongoing ischemic stroke or is at risk of developing an ischemic stroke. If this comparison shows that the subject has a lower level of asymmetry, then it can be detected that the subject does not have an ongoing ischemic stroke or is not at risk of developing an ischemic stroke.

In some embodiments, step 107 comprises a combination of the above embodiments, e.g. a change in asymmetry can be compared to a threshold value, and an ongoing ischemic stroke detected or detecting that the subject is at risk of developing an ischemic stroke based on that comparison.

In some embodiments, if the subject is known to be experiencing or suffering a stroke when the blood flow was measured, then in step 107 if it is determined that the subject is not experiencing or suffering an ischemic stroke, then it can be determined that the subject is experiencing or suffering a haemorrhagic stroke. In some embodiments, the subject can be known to be experiencing or suffering a stroke based on an input provided to the apparatus 2, for example from a user of the apparatus (e.g. a clinician or family member of the subject) or from the subject themselves, e.g. via user interface 10, indicating that the subject is suffering a stroke. Alternatively the subject can be known to be experiencing or suffering a stroke based on a signal received by the apparatus 2 from another apparatus or device that is able to detect ongoing strokes.

In other embodiments, the apparatus 2 may only intended to be used on a subject that is suffering a stroke, in which case the apparatus 2 can be used to make a binary determination between an ischemic stroke and a haemorrhagic stroke (i.e. if an ischemic stroke is not detected) in step 107.

In either of the above embodiments, the method can further comprise providing an output indicating whether the subject has an ongoing ischemic stroke or whether the subject is at risk of developing an ischemic stroke (as appropriate depending on which was determined in step 107).

In further embodiments, the measurements obtained in step 103 can also relate to a time period before the sensory stimulation is applied. Thus, measurements of the blood flow in the left part of the head and/or neck of the subject and measurements of the blood flow in the right part of the head and/or neck of the subject can be obtained before the stimulus is applied.

Preferably these measurements are obtained in a time period immediately preceding the application of the stimulus.

The measurements obtained before the stimulus is applied can be used to normalise the measurements obtained after the stimulus is applied, and provide a more accurate measure of the asymmetry between the left side and the right side. For example, a noisy data source (such as a skin/blood perfusion measurement) can be improved by having a trigger (i.e. the stimulus) that will change the signal in some way.

In particular, a set of measurements of the blood flow for the left side of the head and/or neck obtained after the stimulus is applied can be divided by a set of measurements of the blood flow for the left side of the head and/or neck obtained before the stimulus is applied, to provide a normalised set of measurements of the blood flow for the left side of the head and/or neck. The same can be done for the measurements for the right side. Then, in step 105, the measure of asymmetry can be determined from the normalised set of measurements of the blood flow for the left side of the head and/or neck and the normalised set of measurements of the blood flow for the right side of the head and/or neck. This measure of asymmetry can be determined in the same way as described above for step 105.

In further embodiments, the method in steps 101-105 can be repeated over time to obtain several sets of measurements of the blood flow in the left and right parts of the head and/or neck of the subject following the application of a stimulus, and thus several corresponding measures of asymmetry. In these embodiments, the occurrence of an ischemic stroke or whether the subject is at risk of developing an ischemic stroke can be determined in step 107 by comparing two or more measures of asymmetry to determine whether the asymmetry has increased or decreased between the two measurement times. An increase in the asymmetry can indicate that an ischemic stroke is ongoing or the subject is at risk of developing an ischemic stroke, and vice versa.

In further embodiments, the method in steps 101-105 can be repeated using different stimulus in step 101. For example, a first measure of asymmetry can be obtained using a stimulus that stimulates one side of the brain, and a second measure of asymmetry can be obtained using a stimulus that stimulates the other side (or both sides) of the brain. These measures of asymmetry can be compared, or the highest measure selected, and used in step 107 to determine whether the subject has an ongoing ischemic stroke or whether the subject is at risk of developing an ischemic stroke.

The apparatus 2 and method described above can be put into practice in a number of ways. In a first implementation, the apparatus 2 can be in the form of a smart mirror, or integrated with or associated with a conventional mirror, and the blood flow measurement device 14 can measure blood flow using a camera that can observe a subject in front of the mirror. A stimulus component 12 can be used to apply the sensory trigger, with any measured asymmetry in the blood flow in the face and neck area following the sensory trigger indicating a blockage of the internal carotid artery. As noted above, the use of sensory triggers enables correction of the natural variation in the skin blood flow, which increases the visibility of the asymmetry.

In another implementation, which expands on the first implementation above, the stimulus component 12 can make use of a virtual reality (VR) environment to apply the sensory triggers. The use of VR enables the possibility to focus the sensory trigger to either the left or right side of the brain.

In another implementation, which can be suitable for use in an ambulance, the blood flow measurement device 14 can be either camera-based or non-camera-based (e.g. PPG sensors that can be attached to each ear, or to each side of the face, forehead or neck) for measuring blood flow in the neck and/or face area. The sensory stimulus can be provided using a VR headset, a pair of headphones, a display screen, etc.

There are therefore provided improvements in the detection of an ongoing ischemic stroke or determining whether the subject is at risk of developing an ischemic stroke in a subject.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus for detecting an ongoing ischemic stroke in a subject or detecting whether the subject is at risk of developing an ischemic stroke, the apparatus comprising:
a processing unit configured to:
control a stimulus component to apply a sensory stimulation to the subject to affect the flow of blood to the left part and/or right part of the brain of the subject;
obtain measurements of the blood flow in left and right parts of the head and/or neck of the subject for a time period during and/or after the sensory stimulation is applied to the subject;
determine a measure of asymmetry in the blood flow in the left and right parts of the head and/or neck of the subject from the obtained measurements; and
determine whether the subject has an ongoing ischemic stroke or whether the subject is at risk of developing an ischemic stroke from the determined measure of asymmetry.

2. An apparatus as claimed in claim 1, wherein the processing unit is configured to determine from the measure of asymmetry whether the subject has an ongoing ischemic stroke or is at risk of developing an ischemic stroke by comparing the measure of asymmetry to a threshold value.

3. An apparatus as claimed in claim 1 or 2, wherein the processing unit is configured to determine from the measure of asymmetry whether the subject has an ongoing ischemic stroke or is at risk of developing an ischemic stroke by comparing the determined measure of asymmetry to a previous measure of asymmetry for the subject or to a measure of asymmetry for a population of subjects.

4. An apparatus as claimed in any of claims 1-3, wherein the processing unit is further configured to:
determine that the subject has an ongoing haemorrhagic stroke if it is determined that the subject does not have an ongoing ischemic stroke.

5. An apparatus as claimed in any of claims 1-4, wherein the processing unit is further configured to:
obtain measurements of the blood flow in left and right parts of the head and/or neck of the subject for a time period before the sensory stimulation is applied to the subject; and
normalise the measurements of the blood flow in left and right parts of the head and/or neck of the subject for the time period during and/or after the sensory stimulation is applied using the measurements of the blood flow in left and right parts of the head and/or neck of the subject for the time period before the sensory stimulation is applied;
wherein the processing unit is configured to determine the measure of asymmetry from the normalised measurements of blood flow.

6. An apparatus as claimed in any of claims 1-5, wherein the processing unit is further configured to:
control the stimulus component to apply a second sensory stimulation to the subject to affect the flow of blood to the left part and/or right part of the brain of the subject;
obtain measurements of the blood flow in left and right parts of the head and/or neck of the subject for a time period during and/or after the second sensory stimulation is applied to the subject; and
determine a second measure of asymmetry in the blood flow in the left and right parts of the head and/or neck of the subject from the measurements for the time period during and/or after the second sensory stimulation is applied;
wherein the processing unit is configured to determine whether the subject has an ongoing ischemic stroke or whether the subject is at risk of developing an ischemic stroke from the determined measure of asymmetry and the determined second measure of asymmetry.

7. An apparatus as claimed in claim 6, wherein the sensory stimulation affects the flow of blood to one of the parts of the brain of the subject, and wherein the second sensory stimulation affects the flow of blood to the other part or both parts of the brain of the subject.

8. A method of operating an apparatus to detect an ongoing ischemic stroke in a subject or detect whether the subject is at risk of developing an ischemic stroke, the method comprising:
controlling a stimulus component to apply a sensory stimulation to the subject to affect the flow of blood to the left part and/or right part of the brain of the subject;
obtaining measurements of the blood flow in left and right parts of the head and/or neck of the subject for a time period during and/or after the sensory stimulation is applied to the subject;
determining a measure of asymmetry in the blood flow in the left and right parts of the head and/or neck of the subject from the obtained measurements; and
determining whether the subject has an ongoing ischemic stroke or whether the subject is at risk of developing an ischemic stroke from the determined measure of asymmetry.

9. A method as claimed in claim 8, wherein the method comprises determining from the measure of asymmetry whether the subject has an ongoing ischemic stroke or is at risk of developing an ischemic stroke by comparing the measure of asymmetry to a threshold value.

10. A method as claimed in claim 8 or 9, wherein the method comprises determining from the measure of asymmetry whether the subject has an ongoing ischemic stroke or is at risk of developing an ischemic stroke by comparing the determined measure of asymmetry to a previous measure of asymmetry for the subject or to a measure of asymmetry for a population of subjects.

11. A method as claimed in any of claims 8-10, wherein the method comprises:
determining that the subject has an ongoing haemorrhagic stroke if it is determined that the subject does not have an ongoing ischemic stroke.

12. A method as claimed in any of claims 8-11, wherein the method comprises:
obtaining measurements of the blood flow in left and right parts of the head and/or neck of the subject for a time period before the sensory stimulation is applied to the subject; and
normalising the measurements of the blood flow in left and right parts of the head and/or neck of the subject for the time period during and/or after the sensory stimulation is applied using the measurements of the blood flow in left and right parts of the head and/or neck of the subject for the time period before the sensory stimulation is applied;
wherein the method comprises determining the measure of asymmetry from the normalised measurements of blood flow.

13. A method as claimed in any of claims 8-12, wherein the method further comprises:
controlling the stimulus component to apply a second sensory stimulation to the subject to affect the flow of blood to the left part and/or right part of the brain of the subject;
obtaining measurements of the blood flow in left and right parts of the head and/or neck of the subject for a time period during and/or after the second sensory stimulation is applied to the subject; and
determining a second measure of asymmetry in the blood flow in the left and right parts of the head and/or neck of the subject from the measurements for the time period during and/or after the second sensory stimulation is applied;
wherein the method comprises determining whether the subject has an ongoing ischemic stroke or whether the subject is at risk of developing an ischemic stroke from the determined measures of asymmetry and the determined second measure of asymmetry.

14. A method as claimed in claim 13, wherein the sensory stimulation affects the flow of blood to one of the parts of the brain of the subject, and wherein the second sensory stimulation affects the flow of blood to the other part or both parts of the brain of the subject.

15. A computer program product comprising a computer readable medium having computer readable code embodied therein, the computer readable code being configured such that, on execution by a suitable computer or processor, the computer or processor is caused to perform the method of any of claims 8-14.
